Europäisches Patentamt

European Patent Office

Office européen des brevets

(11) Veröffentlichungsnummer: **0 018 010**
**B1**

(12) **EUROPÄISCHE PATENTSCHRIFT**

(45) Veröffentlichungstag der Patentschrift:
20.04.83

(51) Int. Cl.³: **C 07 D201/16**

(21) Anmeldenummer: **80102128.8**

(22) Anmeldetag: **21.04.80**

(54) **Verfahren zum raschen Abkühlen von dampfförmiges Caprolactam enthaltenden Gasen.**

(30) Priorität: **23.04.79 DE 2916415**

(43) Veröffentlichungstag der Anmeldung:
**29.10.80 Patentblatt 80/22**

(45) Bekanntmachung des Hinweises auf die Patenterteilung: **20.04.83 Patentblatt 83/16**

(84) Benannte Vertragsstaaten:
**BE CH DE FR GB IT LI NL**

(56) Entgegenhaltungen:
**FR A 1 451 825**
**FR A 2 077 349**

**Die Akte enthält technische Angaben, die nach dem Eingang der Anmeldung eingereicht wurden und die nicht in dieser Patentschrift enthalten sind.**

(73) Patentinhaber: **BASF Aktiengesellschaft**
**Carl-Bosch-Strasse 38**
**D-6700 Ludwigshafen (DE)**

(72) Erfinder: **Brand, Uwe**
**Rheingoldstrasse 12**
**D-6841 Rosengarten (DE)**
Erfinder: **Fuchs, Hugo, Dr.**
**Egellstrasse 28**
**D-6700 Ludwigshafen (DE)**
Erfinder: **Schmitz, Ruediger**
**Roemerstrasse 2**
**D-6715 Lambsheim (DE)**

Verfahren zum raschen Abkühlen von dampfförmiges Caprolactam enthaltenden Gasen

Gegenstand der Erfindung ist ein Verfahren zum raschen Abkühlen von dampfförmiges Caprolactam enthaltenden Gasen, die durch katalytische Umlagerung von Cyclohexanonoxim in der Gasphase erhalten wurden, durch Inberührung bringen mit einer fein verteilten Kühlflüssigkeit.

Bei der katalytischen Umlagerung von Cyclohexanonoxim zu Caprolactam in der Gasphase bei Temperaturen von 300 bis 400 °C, in Gegenwart von auf- und abwirbelnden Borsäureträgerkatalysatoren, erhält man Reaktionsgase, die Caprolactam dampfförmig enthalten. Da Caprolactam bei so hohen Temperaturen zersetzlich ist, müssen diese Gase rasch abgekühlt werden, z. B. durch Abschrecken mit Caprolactam oder Wasser. Ein solches Verfahren ist beispielsweise aus der DE-AS 20 03 460 bekannt.

Aus der FR-PS 1 451 825 ist auch ein Verfahren bekannt, bei dem man die den Reaktionsraum verlassenden Gase mit Rohcaprolactam von 205 °C abschreckt.

Es hat sich jedoch herausgestellt, daß es an den Zuführungsstellen des Caprolactam enthaltenden Gasgemisches in der Abkühlvorrichtung zu Abscheidungen kommt, die zu Störungen Anlaß geben.

Es war deshalb die technische Aufgabe gestellt, die Abkühlung von dampfförmiges Caprolactam enthaltenden Gasen so zu gestalten, daß keine Abscheidungen die Störungen verursachen auftreten.

Diese technische Aufgabe wird gelöst in einem Verfahren zum raschen Abkühlen von dampfförmigem Caprolactam enthaltenden Gasen, die durch katalytische Umlagerung von Cyclohexanonoxim in der Gasphase in Gegenwart von Borsäure enthaltenden Trägerkatalysatoren bei einer Temperatur von 330 bis 400° erhalten wurden, durch Abschrecken mit einer fein verteilten Kühlflüssigkeit mit einer Temperatur von 90 bis 200 °C auf eine Temperatur von 100 bis 200°C, wobei man in eine Kühlzone von oben die Kühlflüssigkeit in feiner Verteilung zuführt, im Verlauf der Kühlzone nach unten dampfförmiges Caprolactam enthaltende Gase mit einer Temperatur, die 330 °C nicht unterschreitet, durch Düsenöffnungen mit hoher Geschwindigkeit radial zuführt und das abgekühlte Gemisch ableitet.

Das neue Verfahren hat den Vorteil, daß auf einfache Weise Ablagerungen vermieden werden und somit ein störungsfreies Abschrecken der heißen Gase gewährleistet wird.

Dampfförmiges Caprolactam enthaltende Gase erhält man durch katalytische Umlagerung von Cyclohexanonoxim in der Gasphase in Gegenwart von Borsäure enthaltenden Trägerkatalysatoren. Das Cyclohexanonoxim wird hierbei wasserfrei oder gegebenenfalls wasserhaltig, z. B. mit bis zu 10 % Wassergehalt, gegebenenfalls auch mit einem Zusatz an Borsäure oder Bortrioxid dampfförmig oder flüssig oder in fester Form in die Wirbelschicht geleitet, wo sich der Katalysator auf Reaktionstemperatur befindet. Die Umlagerung wird bei einer Temperatur von 330 bis 400 °C, vorzugsweise 330 bis 370 °C durchgeführt. Das Verfahren läßt sich bei Normaldruck, bei vermindertem Druck oder leichtem Überdruck ausführen. Der Katalysator wird mit Inertgas, wie Kohlendioxid, Argon, Stickstoff, vorzugsweise Stickstoff in wirbelnder Bewegung gehalten. Als Katalysator verwendet man bekannte Katalysatoren, bei denen Bortrioxid oder Borsäure, die unter Reaktionsbedingungen in Bortrioxid übergeht, auf Träger aufgebracht sind. Als Träger sind insbesondere Aluminiumoxid in seinen verschiedenen Modifikationen, ferner Kieselsäure oder Titandioxid sowie Gemische derartiger Oxide untereinander oder Kohle geeignet. Das Gewichtsverhältnis von Bortrioxid zu Träger beträgt im allgemeinen von 1 : 9 bis 1 : 1. Bevorzugte Katalysatoren haben einen Anteil an Bortrioxid von 25 bis 50 Gew.%. Ein geeignetes Verfahren wird beispielsweise beschrieben in der DE-AS 20 03 460. Die so erhaltenen dampfförmiges Caprolactam enthaltenden Gase enthalten in der Regel 5 bis 40 Vol% Caprolactam. Der Rest besteht aus Inertgasen sowie geringen Mengen nicht umgesetztem Cyclohexanonoxim sowie Zersetzungsprodukten des Caprolactams ferner flüchtigen Borsäureverbindungen, die vom Katalysator stammen.

Diese dampfförmiges Caprolactam enthaltende Gase werden durch Inberührungbringen mit einer fein verteilten Kühlflüssigkeit abgeschreckt. Hierbei führt man in eine Kühlzone von oben Kühlflüssigkeit mit einer Temperatur von 90 bis 200 °C in feiner Verteilung zu. Geeignete Kühlflüssigkeiten sind beispielsweise Wasser, geschmolzenes Caprolactam oder niedrigsiedende organische Lösungsmittel, z. B. Kohlenwasserstoffe oder niedere, vorteilhaft mit Wasser nicht mischbare Alkohole. Besonders bevorzugt werden geschmolzenes Caprolactam oder Wasser, insbesondere Caprolactam angewandt. Die Temperatur der zugeführten Kühlflüssigkeit richtet sich nach der Art der angewandten Kühlflüssigkeit. Es versteht sich, daß man Wasser beispielsweise mit einer Temperatur von 90 °C bis unterhalb des Siedepunkts des Wasser anwendet, während es sich für Caprolactam als vorteilhaft erwiesen hat, dieses mit einer Temperatur von 150 bis 200 °C zuzuführen. Die feine Verteilung des Kühlmittels erfolgt beispielsweise durch Einstoffdüsen. Die Zuführung kann an einer oder mehreren Stellen oben in der Kühlzone erfolgen, möglichst so, daß der gesamte Querschnitt der Kühlzone von oben gleichmäßig mit fein verteilter Kühlflüssigkeit beaufschlagt wird.

Im Verlauf der Kühlzone nach unten werden dampfförmiges Caprolactam enthaltende Gase mit einer Temperatur von 330 bis 400 °C durch Düsenöffnungen mit hoher Geschwindigkeit radial zugeführt. Hierbei ist darauf zu achten, daß das abzukühlende Gasgemisch vor Austritt aus den Düsenöffnungen nicht unter 330 °C, vorteilhaft 350 °C abgekühlt wird. Die Düsenöffnungen für die Gaszuführung können außen an der Kühlzone angeordnet sein, so daß das abzukühlende Gas radial nach innen in die Kühlzone strömt. Vorteilhaft sind die Düsenöffnungen über den Umfang der Kühlzone in einer Ebene angeordnet. Besonders bewährt hat es sich, wenn man das abzukühlende Gas coaxial in die Kühlzone zuführt und durch eine ringförmige Düsenöffnung radial von innen nach außen in einer Ebene in die Kühlzone einleitet. Vorteilhaft hält man an den Düsenöffnungen Gasgeschwindigkeiten von 10 bis 25 m/sec ein.

Durch die hohen Strömungsgeschwindigkeiten des eingeführten Gases und die feine Verteilung der Kühlflüssigkeit tritt eine Durchmischung in kürzester Zeit und somit ein Abschrecken des Caprolactams auf eine Temperatur von 100 bis 200 °C ein. Die Abkühlung erfolgt durch Verdampfen von Kühlflüssigkeit. Die angewandte Menge an Kühlflüssigkeit richtet sich nach der Temperatur der zugeführten Flüssigkeit und beträgt in der Regel das 5 bis 60fache der abzukühlenden Caprolactammenge. Verwendet man beispielsweise Caprolactam mit einer Temperatur von 150 bis 200 °C als Kühlflüssigkeit, so hat es sich bewährt, wenn man die 5- bis 25-fache Menge an Caprolactam, bezogen auf abzukühlendes Caprolactam in Gasgemisch zuführt.

Das abgekühlte Gemisch, das nun aus Inertgas flüssigem Caprolactam und gegebenenfalls Kühlflüssigkeit besteht, wird aus der Kühlzone abgeleitet. Es versteht sich, daß das Gasgemisch entsprechend dem Dampfdruck bei den angewandten Temperaturen Caprolactam und gegebenenfalls Kühlflüssigkeit in der Gasphase enthält. Das so erhaltene Gemisch wird zweckmäßig unten in eine Kolonne eingeleitet, die am Kolonnenkopf mit Wasser beaufschlagt wird um eine vollständige Abtrennung des Caprolactams von Inertgas zu erzielen. Eine solche Arbeitsweise ist beispielsweise beschrieben in der DE-AS 20 03 460.

Caprolactam wird für die Herstellung von Polycaprolactam verwendet. Das Verfahren nach der Erfindung sei an folgendem Beispiel veranschaulicht.

Beispiel

In einem Reaktor mit den Dimensionen 5.000 mm Länge und 1.200 mm Durchmesser werden ca. 500 kg eines Katalysators, bestehend aus 58 % Aluminiumoxid und 42 % Bortrioxid der Korngröße 0,3 bis 1,0 mm, vorgelegt. Stündlich werden 560 kg Stickstoff, der durch eine elektrische Heizung auf 360 °C gebracht wurde, über einen Lochboden in den Reaktor geblasen und dadurch der Katalysator zum Wirbeln gebracht. Über 3 Düsen gleichmäßig am Umfang des Reaktors verteilt, werden stündlich 500 kg Cyclohexanonoxim mit einem Wassergehalt von 4,2 Gew.% in die Wirbelschicht zudosiert. Die Eingangstemperatur des zum Wirbeln benötigten Stickstoffs wird in dem Maße gesenkt, wie die Temperatur im Reaktor ansteigt, so daß eine Reaktionstemperatur von 360 °C gehalten wird. Die Reaktionsgase, bestehend aus Caprolactam, Wasser, Inertgasen, und Zersetzungsprodukte des Caprolactams werden zunächst über einen beheizten Zyklon geführt, um mitgerissene Feststoffteilchen abzuscheiden. Anschließend gelangt das Gasgemisch mit einer Temperatur von 360 °C in die Kühlzone. Diese besteht aus einem geschlossenen Raum, in den von unten die Zufuhr der Reaktionsgase über ein Rohr von 200 mm Durchmesser erfolgt. Der obere Teil des Rohres ist derart mit einer Haube versehen, daß zwischen Rohrende und Haube ein Ringspalt von ca. 10 mm entsteht. Das Zulaufrohr ist als Doppelmantelrohr ausgeführt, gefüllt mit einem Isoliermaterial, um einen möglichst geringen Wärmeübergang zur Kühlzone zu verursachen. Zentral über der Haube ist im oberen Teil der Kühlzone eine Einstoffdüse zur Einbringung des als Kühlflüssigkeit dienenden Caprolactam angebracht. Die Düse wird stündlich mit ca. 5.000 kg Caprolactam mit einer Temperatur von 180 °C beaufschlagt. Das abgeschreckte Gas-Flüssigkeitsgemisch entweicht über ein am unteren Teil der Kühlzone befindliches Brüdenrohr und gelangt in den Sumpf einer nachgeschalteten Kondensationskolonne. An dieser werden am Kopf stündlich ca. 150 l Wasser aufgegeben, um eine vollständige Kondensation des Lactams zu erzielen. Über Kopf der Kolonne entweichen die Inertgase, Wasser und leichtsiedende Verunreinigungen. Aus dem Kolonnensumpf werden stündlich die der im Reaktor gebildeten Lactammenge entsprechende Menge Lactam entnommen und aufgearbeitet. Gleichzeitig dient das Lactam des Kolonnensumpfes zur Eindüsung in die Kühlzone.

Dem Reaktor selbst werden stündlich ca. 200 kg Katalysator entnommen und bei ca. 800 °C mit Luft in einem Regenerator aufgearbeitet und in den Reaktor zurückgeführt. Ebenfalls werden dem Katalysator die den Verlusten entsprechende Borsäuremenge in fester Form zugesetzt.

In der Kühlzone konnten über einen Zeitraum von mehreren Wochen keine Verbackungen festgestellt werden.

**Ansprüche**

1. Verfahren zum raschen Abkühlen von dampfförmigem Caprolactam enthaltenden Gasen, die durch katalytische Umlagerung von Cyclohexanonoxim in der Gasphase in Gegenwart von Borsäure enthaltenden Trägerkatalysatoren bei einer Temperatur von 330 bis 400° erhalten wurden, durch Abschrecken mit einer fein verteilten Kühlflüssigkeit mit einer Temperatur von 90 bis 200 °C auf eine Temperatur von 100 bis 200 °C dadurch gekennzeichnet, daß man in eine Kühlzone von oben die Kühlflüssigkeit in feiner Verteilung zuführt, im Verlauf der Kühlzone nach unten dampfförmiges Caprolactam enthaltende Gase mit einer Temperatur die 330 °C nicht unterschreitet durch Düsenöffnungen mit hoher Geschwindigkeit radial zuführt und das abgekühlte Gemisch ableitet.

2. Verfahren nach Anspruch 1, dadurch gekennzeichnet, daß man dampfförmiges Caprolactam enthaltende Gase mit einer Geschwindigkeit von 10 bis 25 m/sec zuführt.

3. Verfahren nach den Ansprüchen 1 und 2, dadurch gekennzeichnet, daß man dampfförmiges Caprolactam enthaltende Gase durch eine ringförmige Düsenöffnung radial von innen nach außen in einer Ebene in die Kühlzone zuführt.

## Claims

1. A process for rapidly cooling gases which contain caprolactam vapour and which have been obtained by catalytic rearrangement of cyclohexanone-oxime in the gas phase in the presence of a supported catalyst containing boric acid at from 330 to 400 °C, by cooling the gases to 100 to 200 °C with a finely divided coolant having a temperature of from 90 to 200 °C, wherein the coolant is fed in finely divided form into a cooling zone from above, gases which have a temperature of not less than 330 °C and contain caprolactam vapour are introduced radially at high speed through nozzle orifices over the downstream portion of the cooling zone, and the cooled mixture is discharged.

2. A process as claimed in claim 1, wherein gases containing caprolactam vapour are introduced at a velocity of from 10 to 25 m/sec.

3. A process as claimed in claims 1 and 2, wherein gases containing caprolactam vapour are fed outwardly into the cooling zone in a radial direction, in one plane, through a ring-shaped nozzle orifice.

## Revendications

1. Procédé de refroidissement rapide de gaz contenant du caprolactame à l'état de vapeur et qui ont été obtenus par transposition catalytique de cyclohexanonoxime en phase gazeuse, en présence de catalyseurs sur support contenant de l'acide borique, à une température de 330 à 400 °C, par refroidissement brusque à une température de 100 à 200 °C, avec un liquide réfrigérant finement distribué, d'une température de 90° à 200 °C, caractérisé par le fait qu'on amène, du dessus, dans une zone de refroidissement, le liquide réfrigérant finement distribué, on fait arriver radialement vers le bas, à vitesse élevée, par des orifices de buses, dans la zone de refroidissement, des gaz contenant du caprolactame à l'état de vapeur, d'une température qui n'est pas inférieure à 330 °C et on décharge le mélange refroidi.

2. Procédé selon la revendication 1, caractérisé par le fait qu'on amène des gaz contenant du caprolactame à l'état de vapeur, d'une vitesse de 10 à 25 m/s.

3. Procédé selon les revendications 1 et 2, caractérisé par le fait qu'on amène des gaz contenant du caprolactame à l'état de vapeur dans la zone de refroidissement, dans un plan, radialement, de l'intérieur vers l'extérieur, par une ouverture de buses annulaire.